# EUROPEAN PATENT APPLICATION

(11) **EP 4 316 841 A1**
(43) Date of publication of application: **07.02.2024**
(21) Application number: 22306179.7
(22) Date of filing: 03.08.2022
(51) Int. Cl.: B33Y 70/00, B29C 64/106, C12M 1/00, C12M 3/00, C12M 1/26

(54) **STERILE ENCLOSURE**

(71) Applicant: SARTORIUS STEDIM FMT SAS, 13400 Aubagne (FR); UNIVERSITE CLAUDE BERNARD - LYON 1, 69100 Villeurbanne (FR); Institut National des Sciences Appliquees de Lyon (Insa Lyon), 69100 Villeurbanne (FR); Ecole Superieure de Chimie, Physique, Electronique de Lyon, 69616 Villeurbanne Cedex (FR); Centre National de la Recherche Scientifique (CNRS), 75016 Paris (FR)
(72) Inventor: GAY, Isabelle, 13124 Peypin (FR); BARBAROUX, Magali, 13112 La Destrousse (FR); DUFOUR, Alexandre, 69006 Lyon (FR); MARQUETTE, Christophe, 69100 Villeurbanne (FR); PETIOT, Emma, 69100 Villeurbanne (FR)
(74) Representative: Derambure Conseil

(57) **Abstract**

The invention concerns a sterile enclosure (2) intended for a printing of a biological three-dimensional structure, the sterile enclosure comprising :
- a rigid top plate (4),
- a rigid base plate (6) comprising a main face (16) intended to receive a biological three-dimensional structure,
- a flexible side wall (8) fixed to the top plate and to the base plate to form an hermetically closed casing (10) delimiting an interior chamber (11),
- a puncturable membrane (22) or an needle adapter arranged on the top plate,
- a stretcher arm (36) articulated to the top plate and to the base plate, the flexible side wall and the stretcher arm being conform to allow the move of the top plate with respect to the base plate in three perpendicular directions (X,Y,Z); the at least one stretcher arm being elastic and pushing the top plate apart from the base plate.

## Description

### Field of the invention

The present invention relates to a sterile enclosure intended for a printing of a biological three-dimensional structure.

### Technical background

It is known to print out biological three-dimensional structures onto a printing platform of a three-dimensional printers. After the structure is formed, the printed components are immersed in a media for a cell culture process. So once printed, the biological three-dimensional structure must be transferred to a separate bioreactor for the cell culture process. However, it is difficult to maintain the sterile conditions during the transfer of the biological three-dimensional structure to the bioreactor.

To avoid this difficulty, it is also known to print out the biological three-dimensional structure in a container adapted to contain and control a culture media and which also comprises a printer or a movable robotic arm equipped with a printing head. After printing, it is no more necessary to transfer the biological three-dimensional structure in a separate bioreactor for the cell culture process.

A drawback of this operating mode is that the container is cumbersome and heavy since it must contain the printer or the movable robotic arm. As a consequence, when the biological three-dimensional structure has been formed, the printer or the movable robotic arm equipped with the printing head cannot be used to print out another structure because the container must be kept hermetically closed during the cell culture process. Due to its size and its mechanical part which are greased, it can be problematic to sterilise the printer or the movable robotic arm and to keep it sterilized. As the container is large and cumbersome, it can be hard to move it to place it in another room during the cell culture process.

### Object and summary of the invention

A first purpose of the present invention is to propose a sterile enclosure which allows an easy displacement of the printing head in at least two perpendicular directions, and in particular in three perpendicular directions without generating stress on the needle and without risk of bending or breaking of the needle. As a consequence, the shape of the biological three-dimensional structure can be better controlled. Another consequence is that the printing platform does not need to be movable along a vertical direction because the sterile enclosure allows an easy move of the printing head along this direction. Accordingly, a less complex printer can be used. In the same way, the printer can use a smaller motor because there less resistance is applied to the robotic arm.

A second purpose of the present invention is to propose a sterile enclosure that is less bulky, cumbersome and heavy. Since the printer or a part of it is not comprised in the sterile enclosure, its size and its weight are significantly reduced.

A third purpose of the present invention is to propose a sterile enclosure that can be easily moved and stored after the printing process.

A fourth purpose of the present invention is to propose a sterile enclosure which allows immediate reuse of the printer after a biological three-dimensional structure has been formed.

A fifth purpose of the present invention is to propose a sterile enclosure which simplifies both the printing of a biological three-dimensional structure and its culture in a single container.

A sixth purpose of the present invention is to propose a method of printing and culturing a three-dimensional biological structure.

These purposes have been reached by a sterile enclosure as defined in the independent claims. Secondary features of the sterile enclosure are set out in the dependent claims.

### Brief description of figures

Fig. 1 is a perspective view of the sterile enclosure according to a first embodiment of the invention;
Fig. 2 is a top view of the top plate of the sterile enclosure illustrated in Figure 1 ;
Fig. 3 is a side view of the top plate of the sterile enclosure illustrated in Figure 1;
Fig. 4 is a perspective view of the septum of the sterile enclosure illustrated in Figure 1;
Fig. 5 is a side view of the base plate of the sterile enclosure illustrated in Figure 1;
Fig. 6 is a side view of a stretcher arm of the sterile enclosure illustrated in Figure 1;
Fig. 7 is a perspective view of the sterile enclosure according to a second embodiment of the invention.

### Detailed description of the invention

The sterile enclosure 2 according to the invention is intended to be used with a printing cartridge or a printing syringe for the printing of a biological three-dimensional structure in the sterile enclosure.

The printing syringe or the printing cartridge may comprise a bioink composition. A bioink composition is a biomaterial capable of forming a hydrogel and a cell population in a controlled three-dimensional shape. An example of bioink is described in WO 2017115056.

Preferably, for the printing operation, the printing syringe or cartridge is mounted on a displaceable printer for example on a displaceable robotic arm. The printing can also be done manually with a printing nozzle.

In the below description, the term "printing" means making a manual deposit or injection of a bioink or making a 3D-printing with a displaceable robotic arm. In the similar manner, the term "printing needle" is used to designate a needle or a deposition nozzle.

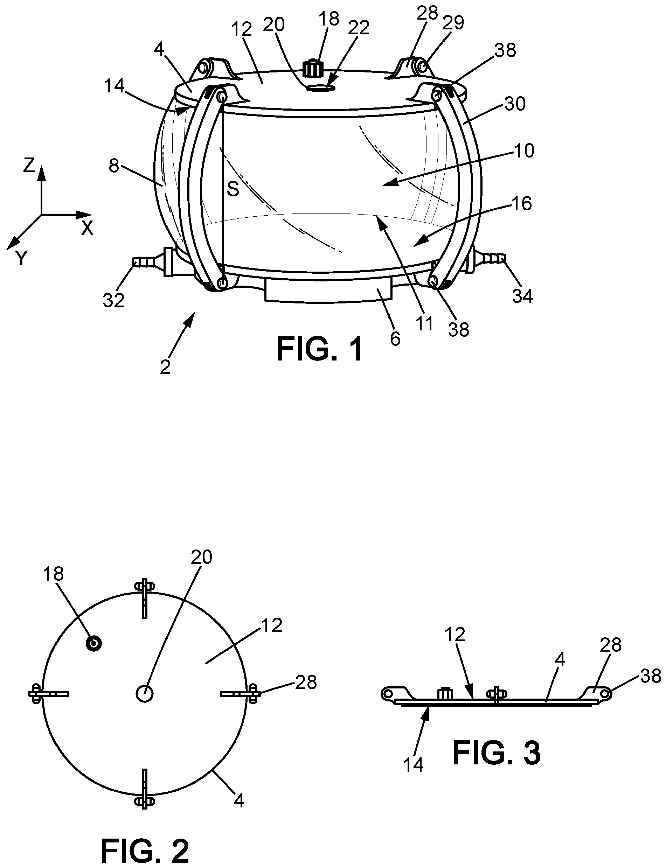

Referring to FIG. 1, the sterile enclosure 2 according to the present invention comprises a rigid top plate 4, a rigid base plate 6, a flexible side wall 8 fixed to the base plate and to the top plate to form a hermetically closed casing 10 delimiting an interior chamber 11.

In the illustrated embodiment, the top plate 4 presents a general shape of a disc having two opposite main faces 12 14. The surface of the main faces are substantially equal to the surface of the upper main face 16 of the base plate.

Advantageously, this large surface of the top plate makes it possible to dispose a great number of aseptic connectors and/or ports.

Alternatively, the main faces 12, 14 of the top plate have a surface which is comprised between 50% to 100% of the surface of the upper main face 16 of the base plate.

In the illustrated embodiment, the top plate 4 comprises a port 18 allowing, for instance, a connection. In the illustrated embodiment this port is a sterile gas vent. This sterile gas vent allows the circulation of air during the printing. So, the pressure in the interior chamber does not change significantly. Alternatively, the top plate 4 can comprise one or several aseptic connector(s), for instance likeOpta^{®} connector commercialised by Sartorius or one or several hose-bard connector(s).

Alternatively, the top plate 4 can comprise one or several aseptic port(s).

The top plate 4 is made of one material among a biocompatible plastic, a metal like a stainless steel and an inorganic material as for example glass and ceramic, a polymer rigid material preferably a polyethylene (PE), a polyethylene terephthalate (PET), Polyurethane (PU), polyvinyl chloride (PVC) or an acide polylactique.

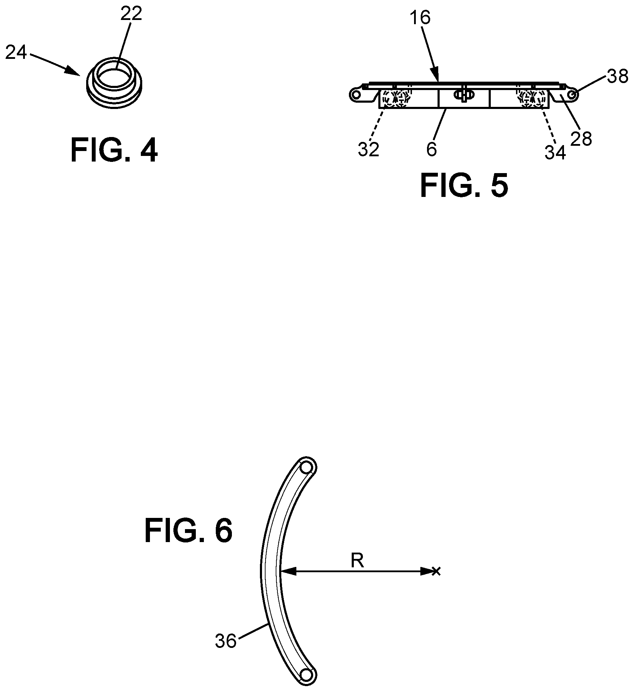

The top plate 4 comprises a hole 20 covered by a puncturable membrane 22. The puncturable membrane 22 is intended to be punctured by a printing needle. It is a septum. One example of septum is illustrated on figure 4. It takes the form of a lid 24. This lid 24 is fixed to the top plate in a sealed manner. It can be glued or fitted in strength into the hole 20. Preferably, the hole 20 is located in the centre of the top plate 4.

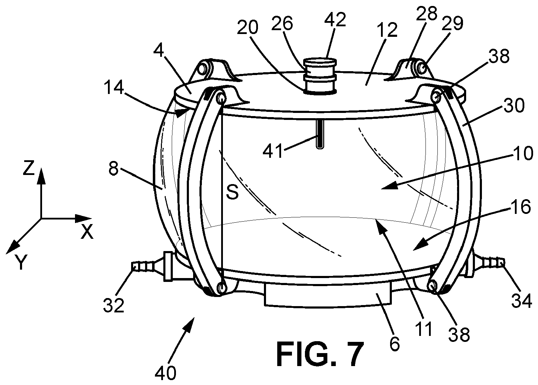

Alternatively, the septum 22 is replaced by a needle adapter 26 holding a printing needle as shown on figure 7.

The top plate 4 comprises further four fasteners 28. The fasteners are regularly angularly spaced around the top plate. Each fastener 28 comprises a pivos shaft 29 on which a stretcher arm 36 is articulated around an upper articulation axis 38.

The base plate 6 comprises a main upper face 16 facing the top plate 4. In the example illustrated, the base plate 6 presents the shape of a disk.

The main upper face 16 is flat. The main upper face 16 of the top plate is intended to receive a biological three-dimensional structure.

The base plate 6 comprises at least one fluid inlet port 32 and at least one fluid outlet port 34 for circulating a cell culture media.

The base plate 6 comprises also four fasteners 28. The fasteners are regularly angularly spaced around the base plate in front of the fasteners 28 of the top plate. Each fastener 28 comprises a pivot shaft 29 on which a stretcher arm 36 is articulated around a lower articulation axis 38.

The base plate 6 is made of one material among a biocompatible plastic, a metal like a stainless steel and an inorganic material as for example glass and ceramic, a polymer rigid material preferably a polyethylene (PE), a polyethylene terephthalate (ET), polyurethane (PU), polyvinyl chloride (PVC) or an acide polylactique.

The flexible side wall 8 is fixed to the top plate 4 and to the base plate 6 to form the container 10.

In the illustrated embodiment, the flexible side wall 8 presents a toroidal shape. Advantageously, this toroidal shape limits the move of the flexible side wall towards the interior chamber during the printing.

Alternatively, the flexible side wall can have a cylinder shape.

Preferably, the flexible side wall 8 is transparent.

The flexible side wall 8 has a hardness shore comprised between A0 to A100 and, preferably, between A0 to A30. The hardness shore is measured by a shore durometer. The flexible side wall 8 presents an elongation comprised between 300% to 2000% and, preferably, between 800% to 1100%. In the present patent application, the term "elongation" is the ratio of the extension of a material to the length of the material prior to stretching.

The flexible wall 8 is made in a biocompatible plastic. Particularly, the flexible wall 8 can be made with one of the following materials:
- Unsaturated rubbers that can be cured by sulfur vulcanization:
   - Natural polyisoprene: cis-1,4-polyisoprene natural rubber (NR) and trans-1,4-polyisoprene gutta-percha
   - Synthetic polyisoprene (IR for isoprene rubber)
   - Polybutadiene (BR for butadiene rubber)
   - Chloroprene rubber (CR), polychloroprene, Neoprene, Baypren etc.
   - Butyl rubber (copolymer of isobutene and isoprene, IIR)
      o Halogenated butyl rubbers (chloro butyl rubber: CIIR; bromo butyl rubber: BIIR)
   - Styrene-butadiene rubber (copolymer of styrene and butadiene, SBR)
   - Nitrile rubber (copolymer of butadiene and acrylonitrile, NBR), also called Buna N rubbers
      o Hydrogenated nitrile rubbers (HNBR) Therban and Zetpol
- Saturated rubbers that cannot be cured by sulfur vulcanization:
   - EPM (ethylene propylene rubber, a copolymer of ethene and propene) and EPDM rubber (ethylene propylene diene rubber, a terpolymer of ethylene, propylene and a diene-component)
   - Epichlorohydrin rubber (ECO)
   - Polyacrylic rubber (ACM, ABR)
   - Silicone rubber (SI, Q, VMQ)
   - Fluorosilicone rubber (FVMQ)
   - Fluoroelastomers (FKM, and FEPM) Viton, Tecnoflon, Fluorel, Aflas and Dai-El
   - Perfluoroelastomers (FFKM) Tecnoflon PFR, Kalrez, Chemraz, Perlast
   - Polyether block amides (PEBA)
   - Chlorosulfonated polyethylene (CSM), (Hypalon)
   - Ethylene-vinyl acetate (EVA)
- Various other types of 4S elastomers:
   - Thermoplastic elastomers (TPE)
   - The proteins resilin and elastin
   - Polysulfide rubber
   - Elastolefin, elastic fiber used in fabric production
   - Poly(dichlorophosphazene), an "inorganic rubber" from hexachlorophosphazene polymerization

Alternatively, the flexible wall 8 can be made with a multilayer film. The multilayer film as described in EP 20945799 could for example be used. For example, the multilayer film comprises a contact layer which is in contact with the medium that fills the container, a barrier layer and an outer layer which is in contact with the external environment. The three layers are connected one to each other with a tie layer. The material can be selected from polyolefins, such as polyethylene. The barrier layer provides a barrier to the passage of gases such as oxygen, carbon dioxide and is typically made from ethylene vinyl alcohol (EVOH). The outer layer could comprise a polyolefin alone or in mixture with a copolymer of ethylene and a-olefin.

The sterile enclosure 2 comprises also four stretcher arms 36 articulated to the top plate 4 and to the base plate 6 around articulation axis 38.

In particular, each stretcher arms 36 is articulated to the pivot shaft 29 of the fasterner 28 of the top plate 4 and to the pivot shaft 29 of the fastener 28 of the base plate 6. The flexible side wall 8 and the stretcher arms 36 are conform to allow the move of the top plate 4 with respect to the base plate 6 in three perpendicular directions (X,Y,Z) while avoiding the contact of the flexible side wall on the biological three-dimensional structure deposited on the upper main face 16, during the printing.

The stretcher arms 36 are elastic. The stretcher arms 36 push the top plate 4 apart from the base plate 6. Advantageously, the stretcher arms 36 make it possible to maintain more or less the top plate 4 parallel to the base plate 6 during the printing.

Advantageously, the stretcher arms 36 allow the enclosure to return to its original shape after printing.

The stretcher arms 36 are curved and have a radius R of curvature comprised between 50 % and 90 % of the length of a vertical segment S passing through an upper articulation axis 38 and a lower articulation axis of a stretcher arm. The greater is the radius of curvature, the greater is the possibility of movement of the top plate 4 with respect to the base plate 6. Preferably, the stretcher arms 36 are located outside of the casing 10.

Preferably, the stretcher arms 36 are made in silicon or in a thermoplastic elastomer.

The stretcher arms 36 have a hardness shore comprised between A0 to A100 and, preferably, between A40 to A100.

The stretcher arms 36 present an elongation comprised between 100% to 2000% and, preferably, between 300% to 600%.

Preferably, the sterile enclosure 2 comprises a sensor to monitor at least one parameter among the Ph, the temperature and the oxygen level. This sensor has not be represented on the figures.

Alternatively, the top plate 4 comprises two or three fasteners or a number of fasteners superior to four. In a similar manner, alternatively, the sterile enclosure comprises two or three stretcher arms or a number of stretcher arms superior to four.

Referring to figure 7, the sterile enclosure 40 according to a second embodiment is similar to the sterile enclosure 2 according to the first embodiment of the present invention at the exception that the puncturable membrane 22 is replaced by a needle adapter 26. The needle adapter 26 is sealed to the top plate 4.

The needle adapter 26 here used is the tip that is usually connected to the open end of a barrel of a medical syringe. The needle adapter 26 comprises a needle hub holding the needle and a connector part 42 configured to be connected to a corresponding connector part of the barrel of the syringe. The connector part 42 and the corresponding connector part are for example a Luer lock connector.

The other technical elements of the second embodiment identical to the technical elements of the first embodiment have been referenced by the same references and will not be described again.

Alternatively, the stretcher arms 36 are located inside of the casing 10.

Alternatively, the top plate and the base plate are square or rectangular and the sterile enclosure comprises four flexible side walls.

The method of printing of a biological three-dimensional structure in the sterile enclosure illustrated on Figure 1 begins with a step during which the printing needle of a 3D printing syringe drills the puncturable membrane 22. The printing needle of the 3D printing syringe is not represented on the figures. The printing needle is introduced into the interior chamber 11 and comes in contact to the main face 16 of the base plate. During this step, the top plate 4 is moved along the vertical direction Z. The top plate 4 is brought closer to the base plate 6. A deposition material in particular bioink is deposited on the main face 16 through the inserted printing needle.

The printing syringe and the top plate 4 is moved with respect to the base plate in a horizontal plane (X, Y) while bioink is deposited into the interior chamber 11. During this step, a biological three-dimensional structure is printed on the upper main face 16 of the top plate. The flexible side wall 8 and the stretcher arms 36 allow the move of the printing needle of the syringe and of the top plate with respect to the base plate while keeping the top plate 4 and the side wall 8 at distance from the already printed biological three-dimensional structure during the printing operation.

The method of printing of a biological three-dimensional structure in the sterile enclosure illustrated on Figure 7 begins with a step during which the printing syringe or the printing cartridge is fixed to the needle adapter a hand the connector part 42. The following steps are identical to the one of the method described above and will not be described again.

## Claims

1. Sterile enclosure (2, 40) intended for a printing of a biological three-dimensional structure, the sterile enclosure (2) comprising :
- a rigid top plate (4),
- a rigid base plate (6) comprising at least one main face (16) intended to receive a biological three-dimensional structure,
- at least one flexible side wall (8) fixed to the top plate and to the base plate (6) to form an hermetically closed casing (10) delimiting an interior chamber (11),
- a puncturable membrane (22) or an needle adapter (26) arranged on the top plate, the puncturable membrane (22) being intended to be punctured by a printing needle,
- at least one stretcher arm (36) articulated to the top plate (4) and to the base plate (6), the flexible side wall (8) and the at least one stretcher arm (36) being conform to allow the move of the top plate (4) with respect to the base plate (6) in three perpendicular directions (X,Y,Z); the at least one stretcher arm (36) being elastic and pushing the top plate (4) apart from the base plate (6).

2. Sterile enclosure (2, 40) according to claim 1, wherein the at least one flexible side wall (8) has a hardness shore comprised between A0 to A100 and, preferably, between A0 to A30.

3. Sterile enclosure (2, 40) according to any of claims 1 and 2, wherein the at least one flexible side wall (8) presents an elongation comprised between 300% to 2000% and, preferably, between 800% to 1100%.

4. Sterile enclosure (2 40) according to any of claims 1 to 3, wherein the at least one stretcher arm (36) has a hardness shore comprised between A0 to A100 and, preferably, between A40 to A100.

5. Sterile enclosure (2 40) according to any of claims 1 to 4, wherein the at least one stretcher arm (36) presents an elongation comprised between 100% to 2000% and, preferably, between 300% to 600%.

6. Sterile enclosure (2, 40) according to any of claims 1 to 5, wherein the at least one stretcher arm (36) is articulated to the top plate (4) and to the base plate (6) around articulation axis (38), the at least one stretcher arm (36) being curved and having a radius of curvature (R) comprised between 50 % and 90 % of the length of a segment (S) passing through and delimited by the articulation axis (38) of the at least one stretcher arm.

7. Sterile enclosure (2, 40) according to any of claims 1 to 6, wherein the at least one stretcher arm (36) is made in silicon or in a thermoplastic elastomer.

8. Sterile enclosure (2, 40) according to any of claims 1 to 7, wherein the flexible side wall (8) is made in a biocompatible plastic among a multiplayer film, a polydimethylsiloxane, a silicone, an unsaturated rubbers that can be cured by sulfur vulcanization, a saturated rubbers that cannot be cured by sulfur vulcanization, a thermoplastic elastomers (TPE), proteins resilin and elastin, a polysulfide rubber, an elastolefin, an elastic fiber used in fabric production, a poly(dichlorophosphazene) and an inorganic rubber from hexachlorophosphazene polymerization.

9. Sterile enclosure (2, 40) according to any of claims 1 to 8, wherein the base plate (6) or the top plate (4) are made of one material among a biocompatible plastic, a metal and an inorganic material, a polymer rigid material preferably a polyethylene (PE).

10. Sterile enclosure (2, 40) according to any of claims 1 to 9, wherein the top plate (4) comprises at least one port (18) and/or one aseptic connector.

11. Sterile enclosure (2, 40) according to any of claims 1 to 10, wherein the top plate (4) and the base plate (6) present the shape of a disk and the flexible side wall (8) is cylindrical or toroidal.

12. Sterile enclosure (2, 40) according to any of claims 1 to 11, wherein the flexible side wall (8) is transparent.

13. Sterile enclosure (2 40) according to any of claims 1 to 12, which comprises at least one sensor to monitor at least one parameter among the Ph, the temperature and the oxygen level.

14. Sterile enclosure (2, 40) according to any of claims 1 to 13, wherein the at least one stretcher arm (36) extends outside of the casing (10).

15. Sterile enclosure (2, 40) according to any of claims 1 to 14, wherein the surface of at least one main face (12, 14) of the top plate (4) is comprised between 50% to 100% of the surface of at least one main face (16) of the base plate (6).

16. Sterile enclosure (2, 40) according to any of claims 1 to 15, wherein the base plate (6) comprises at least one fluid inlet port (32) and at least fluid outlet port (34) for circulating a cell culture media.
